# EUROPÄISCHE PATENTANMELDUNG

(11) **EP 0 599 141 A2**
(43) Veröffentlichungstag der Anmeldung: **01.06.1994**
(21) Anmeldenummer: 93118265.3
(22) Anmeldetag: 11.11.1993
(51) Int. Cl.: G01N 33/94, C07K 15/28, G01N 33/58

(54) **Mittel und Verfahren zur immunologischen Bestimmung von Amiodaron und dessen Metaboliten**

(30) Priorität: 24.11.1992 DE 4239430
(71) Anmelder: MERCK PATENT GmbH, D-64293 Darmstadt (DE)
(72) Erfinder: Heubner, Arnulf, Dr., D-55131 Mainz (DE); Schwarz, Michael, Dr., D-64521 Gross-Gerau (DE); Jalalian, Mohammad, Dr., D-64354 Reinheim 5 (DE); Reckmann, Bernd, Dr., D-64342 Seeheim (DE)

(57) **Zusammenfassung**

Die Erfindung betrifft Mittel und Verfahren zur immunologischen Bestimmung von Amiodaron und dessen Metaboliten in Körperflüssigkeiten sowie die für eine Bestimmung erforderlichen Tracer, Immunogene und Antikörper und deren Herstellung.

## Beschreibung

Die Erfindung betrifft Mittel und Verfahren zur immunologischen Bestimmung von Amiodaron und dessen Metaboliten in Körperflüssigkeiten oder in vorbehandelten Körperflüssigkeiten sowie die für eine Bestimmung erforderlichen Tracer, Immunogene und Antikörper und deren Herstellung.

Arzneimittel werden häufig unter- oder überdosiert verabreicht. Diese Erfahrung erfordert in zunehmendem Maße die Messung der Serumkonzentration der Arzneimittel. Dies ist umso wichtiger, wenn diese Arzneimittel einen engen therapeutischen Bereich haben und Komplikationen bei Überschreiten dieses Bereiches stark zunehmen. Besonders geeignete und schnelle Bestimmungsverfahren sind Immunoassays, insbesondere Fluoreszenzpolarisations-Immunoassays oder Enzym-Immunoassays. Amiodaron (2-Butyl-3-[3,5-diiod-4-(β-diethylaminoethoxy)-benzoyl]benzofuran) besitzt antianginale und antiarrhytmische Eigenschaften. Es wird häufig bei ventrikulären und supraventrikulären Arrhythmien eingesetzt. Amiodaron wird teilweise zum therapeutisch aktiven Desethylamiodaron metabolisiert. Nicht selten kommt es dosisabhängig durch die Einnahme zu Nebenwirkungen wie Fotosensitivität, Verfärbung der Haut, Mikroablagerungen auf der Cornea und Störung der Schilddrüsenfunktion. Deshalb wird in der Literatur empfohlen, Serumspiegel von 1-2 mg/l nicht zu überschreiten.

Die Serumspiegel korrelieren mit der verabreichten Dosis, mit den morphologischen Veränderungen in einigen Geweben und den toxischen Nebenwirkungen. Die Bestimmung der Serumspiegel von Amiodaron erfolgt bisher durch verschiedene HPLC-Methoden. Diese Methoden erfordern jedoch eine Probenvorbehandlung (Eiweißfällung) und ein aufwendiges analytisches Instrumentarium; sie sind darüberhinaus sehr zeitaufwendig.

Für die Herstellung eines Immunoassays benötigt man bekanntlich Tracer, Immunogene und spezifische Antikörper. Nach den im Stand der Technik beschriebenen Methoden wird ein Tracer durch Kopplung eines zu bestimmenden Analyten oder dessen Metaboliten mit einem Marker (Chromogen, Fluorogen, Enzym, radioaktive Markierung) hergestellt. Ein Immunogen wird durch Kopplung eines Haptens oder Haptenderivats mit einem Protein hergestellt. Die Kopplungsreaktionen erfolgen gewünschtenfalls mit Hilfe eines Crosslinkers oder Spacers. Immunogene werden in einem wäßrigen System, vorzugsweise in einem Puffersystem hergestellt und anschließend aufgereinigt. Aufgrund der sehr schlechten Löslichkeit von Amiodaron und dessen Derivaten in wäßriger Lösung ist es nicht möglich, die entsprechenden Immunogene mit Hilfe der bekannten Methoden herzustellen.

Der Erfindung liegt die Aufgabe zugrunde, Mittel und Verfahren zur Verfügung zu stellen, die es erlauben, Amiodaron und dessen Metabolite ohne großen analytischen Aufwand schnell und reproduzierbar zu bestimmen.

Gegenstand der Erfindung sind Verbindungen der allgemeinen Formel (I) 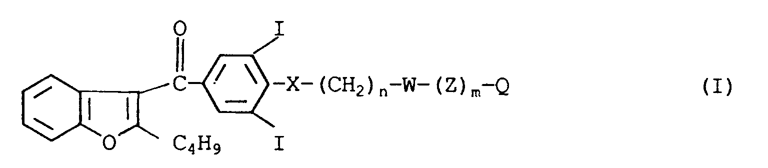
worin
- X: = 0, S, NR¹
- W: = CO, NR¹, S, O
- Z: = ein Crosslinker oder Spacer
- Q: = ein Marker oder ein Trägerprotein
- R¹: = H, Alkyl mit 1-6 C-Atomen
- n: = 1-10
- m: = 0 oder 1
bedeuten.

Ein weiterer Gegenstand der Erfindung sind Antikörper, die durch Immunisierung mit einem Immunogen der allgemeinen Formel (I) erhältlich sind, worin Q ein Trägerprotein bedeutet.

Ferner betrifft die Erfindung ein Mittel zur immunologischen Bestimmung von Amiodaron und dessen Metaboliten in Körperflüssigkeiten, das dadurch gekennzeichnet ist, daß es
a) ein markiertes Amiodaronderivat der allgemeinen Formel (I), worin Q ein Marker bedeutet,
b) Antikörper, die Amiodaron, dessen Metabolite und das markierte Amiodaronderivat binden können und
c) gegebenenfalls ein Enzymsubstrat enthält.

Ein weiterer Gegenstand der Erfindung ist ein Verfahren zur immunologischen Bestimmung von Amiodaron und dessen Metaboliten in Körperflüssigkeiten durch Inkubation der zu untersuchenden Probe mit einem markierten Amiodaronderivat der allgemeinen Formel (I), worin Q ein Marker bedeutet, und Antikörpern, die Amiodaron, dessen Metaboliten und das markierte Amiodaronderivat binden können sowie gegebenenfalls mit einem Enzymsubstrat und Messung der Fluoreszenz oder der Enzymaktivität.

Der Tracer ist bei einem Fluoreszenzpolarisations-Immunoassay mit einerfluoreszierenden Verbindung, bei einem Enzym-Immunoassay mit einem Enzym markiert. Er besteht aus einem Amiodaronderivat der allgemeinen Formel II, 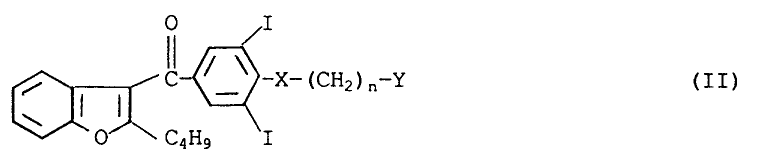
worin
- X: = 0, S, NR¹
- Y: = NHR¹, COR², SH, OH, Halogen, CN
- R¹: = H, Alkyl mit 1-6 C-Atomen
- R²: = OH, Halogen, NHR¹, OR¹
- n: = 1-10
bedeuten,
das gewünschtenfalls mit Hilfe eines Crosslinkers oder Spacers mit einem Fluoreszeinderivat bzw. mit einem Enzym verbunden ist.

Die Herstellung der Verbindungen der allgemeinen Formel (II) erfolgt in der Weise, daß man z.B. ausgehend von 2-Butyl-3-(4-hydroxyphenyl-carbonyl)-benzofuran, den Phenylring in 3- und 5-Stellung iodiert und anschließend mit Bromcarbonsäureethylester in 4-Stellung alkyliert. Die alkalische Verseifung führt zu 2-Butyl-3-[3,5-diiod-4-(carboxyalkyloxy)-benzoyl]-benzofuran. Aus der freien Carbonsäure lassen sich nach bekannten Methoden die entsprechenden Derivate herstellen, die mit einem Crosslinker oder Spacer oder auch direkt mit einem Marker oder Trägerprotein umgesetzt werden können.

Alkyl bedeutet Methyl, Ethyl, Propyl, Butyl, Pentyl, Hexyl und deren Isomere, vorzugsweise Ethyl, Propyl, Isopropyl, n-Butyl, sec.-Butyl oder tert.-Butyl. Halogen ist vorzugsweise Chlor und Brom. Die erfindungsgemäß bevorzugten Verbindungen der allgemeinen Formel (II) sind die Carbonsäureester mit 2 bis 6 C-Atomen in der Alkylgruppe, insbesondere die Propylen- und Butylenverbindungen.

Geeignete Fluoreszeinderivate sind z.B. alle Aminofluoreszeine, Carboxyfluoreszein, Fluoreszeinisothiocyanat, 2,4-Dichlor-1,3,5-triazin-2-ylamino-fluoreszein, 4-Chlor-6-methoxy-1,3,5-triazin-2-ylamino-fluoreszein, 2-Iodoacetamidofluoreszein, 2-Bromoacetamidofluoreszein, Fluoreszeinthiocarbamylethylendiamin, 5-Aminomethylfluoreszein, vorzugsweise 4-Aminofluoreszein.

Geeignete Crosslinker oder Spacer für die Kopplung des Fluoreszeinderivats an das Amiodaronderivat der allgemeinen Formel (II) sind aus der Literatur bekannt. Bevorzugte Crosslinker für das erfindungsgemäße Verfahren sind z.B. 1-Ethyl-3-dimethylaminopropylcarbodiimid-hydrochlorid (EDC), N-Hydroxysuccinimid (NHS), N-(3-Dimethylaminopropyl)-N'-ethylcarbodiimid-hydrochlorid (ECD), Dicyclohexylcarbodiimid (DCC).

Die Herstellung der Tracer erfolgt nach bekannten Methoden in organischen Lösungsmitteln wie Dimethylformamid, Dimethylsulfoxid, Acetonitril, Methanol, Pyridin usw. bei Raumtemperatur.

Die für die erfindungsgemäße Bestimmung von Amiodaron erforderlichen spezifischen Antikörper werden durch Immunisierung mit einem Immunogen aus einem Amiodaronderivat der allgemeinen Formel (II), das gewünschtenfalls über einen Crosslinker oder Spacer an ein Trägerprotein kovalent gekoppelt ist, erhalten. Als Crosslinker eignen sich die oben für die Tracerherstellung genannten Verbindungen. Als Trägerproteine werden z.B. Keyhole Limpet Hemocyan in (KLH), Rinderserumalbumin (BSA), IgG, Ovalbumin, Thyroglobulin oder Lactalbumin verwendet.

Wie bereits erwähnt, ist es aufgrund der schlechten Löslichkeit von Amiodaron und dessen Derivaten nicht möglich, die erforderlichen Immunogene auf übliche Weise in wäßriger Lösung herzustellen. Sie lösen sich vor allem in den üblichen organischen Lösungsmitteln wie Dimethylsulfoxid, Dimethylformamid, Ethanol usw., was jedoch zur irreversiblen Denatuierung des Trägerproteins führt. Überraschenderweise wurde gefunden, daß Amiodaron und dessen Derivate sich in Diethylenglycolmonoalkylether, Diethylenglycoldialkylether, 1,3-Dimethyl-2-imidazolidinon, 3-Butyrolacton, Glycerinformal, 2,2-Dimethyl-4-hydroxymethyl-1,3-dioxolan sehr gut lösen. Auf diese Weise ist es damit erstmals gelungen, ein Immunogen aus einem Amiodaron der allgemeinen Formel (II) und einem Trägerprotein herzustellen. Die Immunogensynthese verläuft ansonsten nach in der Literatur beschriebenen Methoden (Ann. Clin. Biochem. 23, 37 (1986)).

Zur Herstellung der erforderlichen Antikörper wird das aufgereinigte Immunogen in üblicher Weise in zwei- bis dreiwöchigem Abstand in bestimmte Tiere (Schafe, Ziegen, Mäuse, Kaninchen) injiziert. Nach 3 bis 6 Immunisierungen haben sich die gewünschten Antikörper gebildet. Die Aufarbeitung und Reinigung der Antikörper erfolgt nach bekannten Methoden.

Der Tracer für den Enzymimmunoassay wird aus einem Derivat der allgemeinen Formel (II) und einem Markerenzym, z.B. Alkalische Phosphatase, Glucosidase, Galactosidase, Meerrettichperoxidase hergestellt. Die Synthese erfolgt in Gegenwart der für die Immunogensynthese genannten Lösungsmitteln, wobei auf den Crosslinker auch verzichtet werden kann.

Das erfindungsmäßige Verfahren zur Bestimmung von Amiodaron und dessen Metaboliten, insbesondere Desethylamiodaron, wird so durchgeführt, daß die mit Puffer verdünnte Probelösung mit dem Tracer und dem Antikörper gemischt werden. Nach einer bestimmten Inkubationszeit wird die Konzentration von Amiodaron und dessen Metaboliten direkt in der Reaktionslösung bestimmt.

Im Falle des Fluoreszenzpolarisations-Immunoassays wird der Tracer z.B. mit blauem polarisiertem Licht (488 nm) angeregt; die Polarisation des grünen ausgestrahlten Lichts (532 nm) hängt reziprok von der Größe, der Gestalt und der Bewegung des Tracers ab. Die Polarisation des ausgestrahlten Lichts steigt an, wenn der Tracer an den spezifischen Antikörper gebunden vorliegt. Fluoreszenzpolarisations-Immunoassays sind kompetitive Immunoassays, d.h. Tracer und Analyt konkurrieren um die Bindungsstelle am Antikörper. Das Signal der Fluoreszenzpolarisation hängt umgekehrt proportional von der Konzentration des Analyten in der Probe ab. Ist z.B. die Konzentration des Analyten in der Probe hoch, bindet nur eine kleine Menge des Tracers an den Antikörper und das Signal der Fluoreszenzpolarisation ist niedrig.

Im Falle des kompetitiven Enzym-Immunoassays wird der spezifische Antikörper auf einen festen Träger, z.B. auf Mikrotiterplatten, in Röhrchen oder auf magnetischen Partikeln immobilisiert und mit der Probelösung und dem Enzymkonjugat inkubiert. Anschließend wird der Träger gewaschen, mit dem entsprechenden Enzymsubstrat versetzt und die Konzentration des Analyten photometrisch bestimmt.

### Beispiel 1

### Herstellung von 2-Butyl-3-[3,5-diiod-4-(3-carboxypropyloxy)-benzoyl]benzofuran

In 200 ml Acetonitril werden 32,8 g (60 mmol) 2-Butyl-3-[3,5-diiodo-4-hydroxy-benzoyl]benzofuran und 13,8 g (100 mmol) wasserfreies Kaliumcarbonat vorgelegt. 17,6 g (90 mmol) Ethyl-4-brombutyrat werden bei Raumtemperatur innerhalb von 30 Minuten zugetropft und die Suspension anschließend 14 h unter Rückfluß erhitzt. Nach dem Abkühlen auf Raumtemperatur wird die Reaktionslösung mit 200 ml destilliertem Wasser versetzt und 3 mal mit 100 ml MTB-Ether extrahiert. Die vereinten organischen Extrakte werden getrocknet und zur Trockene eingeengt. Der Rückstand, der aus 2-Butyl-3-[3,5-diiodo-4-(3-ethoxycarbonyl-propyloxy)benzoyl)benzofuran besteht, wird in 100 ml Isopropanol vorgelegt und eine Lösung von 39,6 g (600 mmol) 85%igem Kaliumhydroxid in 400 ml destilliertem Wasser zugegeben. Die Suspension wird auf etwa 60 °C erwärmt, bis sich eine klare Lösung bildet. Die auf Raumtemperatur abgekühlte Lösung wird mit 100 ml 1N Salzsäure versetzt, mit 32%iger Salzsäure unter Kühlung auf einen pH-Wert von 3 eingestellt und mit 3 mal 200 ml MTB-Ether extrahiert. Die vereinten organischen, getrockneten Extrakte werden in 50 ml Dichlormethan aufgenommen und für mehrere Stunden bei 0 °C belassen. Die ausgefallenen hellbraunen Kristalle werden mit weiteren 50 ml Dichlormethan verrührt. Nach Absaugen, Waschen und Trocknen erhält man 5,7 g (20 %) fast farblose Kristalle von 2-Butyl-3-[3,5-diiodo-4-(3-carboxy-propyloxy)-benzoyl]benzofuran, welche gewünschtenfalls über einer Kieselgelsäule (MTB-Ether/Methanol = 9:1) weiter aufgereinigt werden können.

### Beispiel 2

### Herstellung eines Immunogens mit KLH

230 mg (0,36 mmol) des gemäß Beispiel 1 hergestellten Amiodaronderivats werden in 1,5 ml Diethylenglycolmonoethylether gelöst. Ebenso werden 50 mg (0,72 µmol) KLH in 25 ml Diethylenglycolmonoethylether zur Lösung gebracht. Die beiden Lösungen werden miteinander vermischt und 3 Stunden bei Raumtemperatur gerührt. Nach Beendigung der Reaktion wird der Ansatz gegen demineralisiertes Wasser bei 4 °C dialysiert. Nach der Dialyse wird die Lösung, welche das Konjugat aus Amiodaronderivat und KLH enthält, zur Immunisierung verwendet.

### Beispiel 3

### Herstellung eines Immunogens mit BSA

Zu der Lösung des Amiodaronderivats gemäß Beispiel 1 werden 500 µl demineralisiertes Wasser, in dem 57 mg (0,3 mmol) EDC und 38 mg (0,33 mmol) NHS gelöst wurden, gegeben. Diese Lösung gibt man zu einer Lösung von 50 mg BSA in 25 ml Diethylenglycolmonoethylether. Anschließend wird gemäß Beispiel 2 verfahren. Man erhält ein Immunkonjugat aus dem Amiodaronderivat gemäß Beispiel 1, den genannten Crosslinkern und BSA.

### Beispiel 4

### Herstellung eines fluoreszenzmarkierten Tracers

11 mg (5,6 mmol) des Amiodaronderivats nach Beispiel 1 werden mit 15 mg (19,6 mmol) EDC und 3 mg (6,5 mmol) NHS in 1 ml Dimethylformamid gelöst und 30 Minuten bei Raumtemperatur gerührt.

10 mg (5,1 mmol) Fluoreszein-thiocarbamylethylendiamin werden in 3 ml Dimethylformamid gelöst und 10 Minuten bei Raumtemperatur gerührt. Anschließend wird diese Lösung tropfenweise zur ersten Lösung hinzugegeben und der gesamte Ansatz wird bei Raumtemperatur über Nacht gerührt. Die Isolierung und Aufreinigung des Tracers erfolgt mit Hilfe der Dünnschichtchromatographie. Laufmittel: Chloroform + Methanol 8+1.

### Beispiel 5

### Herstellung eines enzymmarkierten Tracers

57 mg EDC und 38 mg NHS werden in 500 µl demineralisiertem Wasser gelöst und mit einer Lösung des Amiodaronderivats nach Beispiel 1 (230 mg) in 1,5 ml Diethylenglycolmonoethylether versetzt. 50 mg Meerrettichperoxidase werden in 25 ml Diethylenglycolmonoethylether gelöst. Beide Lösungen werden miteinander vermischt und 3 Stunden bei Raumtemperatur gerührt. Nach Beendigung der Reaktion wird der gesamte Ansatz gegen Phosphatpuffer bei 4 °C dialysiert.

### Beispiel 6

### Herstellung des Antikörpers gegen Amiodaron und dessen Metaboliten

0,8 mg des Immunogens nach Beispiel 3 werden mit einem Adjuvans (Komplettes oder inkomplettes Freundsches Adjuvans, Aluminiumhydroxid, Wasser-Öl-Emulsion oder Muramyldipeptid) emulgiert. Mit dieser Emulsion wird das Tier zweimal im Abstand von 21 Tagen immunisiert. Danach wird es 4-12 mal mit einer Emulsion aus 0,8 mg Amiodaron-BSA mit inkomplettem Freundschen Adjuvans im Abstand von 21 Tagen immunisiert.

### Beispiel 7

### Fluoreszenzpolarisations-Immunoassay

Humanserum wird mit einem üblichen Fällungsreagenz enteiweißt und der Fällungsüberstand bzw. der verdünnte Fällungsüberstand wird im Test eingesetzt. Die Antikörper aus Schaf werden je nach Titer zwischen 1:5 und 1:1000 mit Trispuffer, pH-Wert 7,5, verdünnt. Der Test wird nach folgendem Pipettierschemna durchgeführt:
1000 µl Systempuffer (Trispuffer, pH 7,5),
50 µl Antikörperlösung,
150 µl Fällungsüberstnad bzw. verdünnter Fallungsüberstand bzw. Kalibrator.

Nach der Messung des Probenleerwertes kommen 50 µl Tracer hinzu, der auf eine Konzentration von 100 nmol/l eingestellt ist. Nach einer Inkubation von 6 Minuten bei 37 °C wird die Amiodaron-Konzentration gemessen. Das System wird mit 6 Standards kalibriert; diese auf Humanserum basierenden Standards enthalten Amiodaron in Konzentrationen von 0-6 mg/l.

Gegebenenfalls kann der Test auch ohne vorausgehende Enteiweißung durchgeführt werden.

### Beispiel 8

### Enzym-Immunoassay

Die Bestimmung erfolgt nach dem Prinzip des kompetitiven Enzymimmunoassays bei Raumtemperatur. Es werden die gleichen Standards wie in Beispiel 7 verwendet. Der Antikörper wird auf der Mikrotiterplatte oder in Röhrchen direkt oder indirekt (über anti-Schaf-IgG) immobilisiert. Unter Verwendung der Mikrotiterplatte werden pipettiert:
50 µl verdünnter Fällungsüberstand bzw. verdünnter Kalibrator (1:50 verdünnt),
50 µl Enzymkonjugat (Meerrettich-Peroxidase) aus Beispiel 5.

Es wird für 1 Stunde bei Raumtemperatur inkubiert.

Nach dem Waschen der Inkubationsgefäße kommen 100 µl Substrat (2,2'-Azino-di-(3-ethyl-benzthiazolinsulfonsäure(6))-diammoniumsalz) hinzu.

Es wird weitere 15 Minuten lang inkubiert. Anschließend wird die optische Dichte bei 405 nm gemessen.

## Patentansprüche

1. Verbindungen der allgemeinen Formel (I) 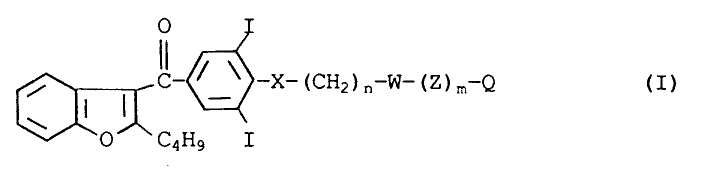 worin
X = 0, S, NR¹
W = CO, NR¹, S, O
Z = ein Crosslinker oder Spacer
Q = ein Marker oder ein Trägerprotein
R¹ = H, Alkyl mit 1-6 C-Atomen
n = 1-10,
m = 0 oder 1
bedeuten.

2. Antikörper erhältlich durch Immunisierung mit einem Immunogen der allgemeinen Formel (I), worin X, Z, R¹, n und m die angegebene Bedeutung haben und Q ein Trägerprotein ist.

3. Mittel zur immunologischen Bestimmung von Amiodaron und dessen Metaboliten in Körperflüssigkeiten, dadurch gekennzeichnet, daß es
a) ein markiertes Amiodaronderivat der allgemeinen Formel (I), worin Q ein Marker bedeutet,
b) Antikörper, die Amiodaron, dessen Metaboliten und das markierte Amiodaronderivat binden können und
c) gegebenenfalls ein Enzymsubstrat enthält.

4. Mittel nach Anspruch 3, dadurch gekennzeichnet, daß das markierte Amiodaronderivat aus einer über einen Crosslinker oder Spacer mit einem Marker verbundenen Verbindung der allgemeinen Formel (I) besteht.

5. Mittel nach Anspruch 3, dadurch gekennzeichnet, daß das Immunogen zur Herstellung der Antikörper aus einer über einen Crosslinker oder Spacer mit einem Trägerprotein verbundenen Verbindung der allgemeinen Formel (I) besteht.

6. Verfahren zur immunologischen Bestimmung von Amiodaron und dessen Mataboliten in Körperflüssigkeiten durch Inkubation der zu untersuchenden Probe mit einem markierten Amiodaronderivat der allgemeinen Formel (I), worin Q ein Marker bedeutet, und Antikörpern, die Amiodaron, dessen Metabolite und das markierte Amiodaronderivat binden können sowie gegebenenfalls mit einem Enzymsubstrat und Messung der Fluoreszenz oder der Enzymaktivität.

7. Verfahren nach Anspruch 6, dadurch gekennzeichnet, daß als Amiodaronderivat eine über einen Crosslinker oder Spacer mit einem Marker verbundene Verbindung der allgemeinen Formel (II). 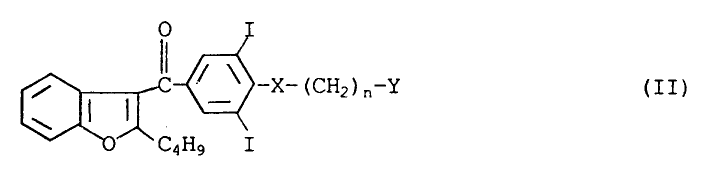 worin
X = 0, S, NR¹
Y = NHR¹, COR², SH, OH, Halogen, CN
R¹ = H, Alkyl mit 1-6 C-Atomen
R² = OH, Halogen, NHR¹, OR¹
n = 1-10,
bedeuten, eingesetzt wird.

8. Verfahren nach Anspruch 6, dadurch gekennzeichnet, daß die Antikörper mit Hilfe von über einen Crosslinker oder Spacer mit einem Trägerprotein verbundenen Verbindung der allgemeinen Formel (II) als Immunogen hergestellt werden.

9. Verfahren nach Anspruch 8, dadurch gekennzeichnet, daß die Herstellung des Immunogens in Gegenwart von Diethylenglycolmonoalkylether, Diethylenglycoldialkylether, 1,3-Dimethyl-2-imidazolidinon, 3-Butyrolacton, Glycerinformal, 2,2-Dimethyl-4-hydroxymethyl-1,3-dioxalan, durchgeführt wird.

10. Die Verbindungen der allgemeinen Formel (II) 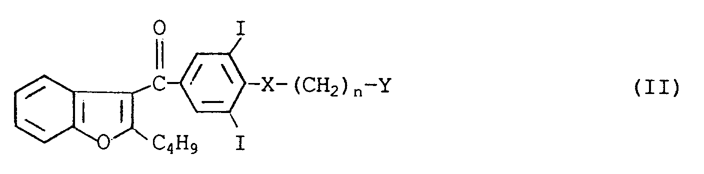 worin X, Y und n die oben angegebene Bedeutung haben.
